(19) Europäisches Patentamt
European Patent Office
Office européen des brevets

(11) Publication number: **0 300 423 A2**

(12) **EUROPEAN PATENT APPLICATION**

(21) Application number: 88111597.6

(51) Int. Cl.4: C07D 501/36 , A61K 31/545

(22) Date of filing: 19.07.88

(30) Priority: 21.07.87 JP 182802/87
21.07.87 JP 182803/87

(43) Date of publication of application:
25.01.89 Bulletin 89/04

(84) Designated Contracting States:
AT BE CH DE ES FR GB IT LI NL SE

(71) Applicant: Kyoto Pharmaceutical Industries, Ltd.
38, Nishinokyo Tsukinowa-cho Nakakyo-ku
Kyoto-shi Kyoto 604(JP)

(72) Inventor: Nishizawa, Susumu
Heim-Fushimi C-610 1, Yamaden Shimomisu
Yokooji
Fushimi-ku Kyoto-shi Kyoto(JP)
Inventor: Muro, Hiroyuki
Ohama 283 Biwa-cho
Higashiazai-gun Shiga(JP)
Inventor: Kasai, Masayasu
6-3-401, Kitakawaramachi 2-chome
Kameoka-shi Kyoto(JP)
Inventor: Hatano, Satoru
6-1-403, Kitakawaramachi 2-chome
Kameoka-shi Kyoto(JP)
Inventor: Kamiya, Syouzi 202, Nakagawa
Co.op. 5
50-39, Kamikatsura Sannomiya-cho
Nishikyo-ku
Kyoto-shi Kyoto(JP)
Inventor: Kakeya, Nobuharu
10-16, Takadai 3-chome
Nagaokakyo-shi Kyoto(JP)
Inventor: Kitao, Kazuhiko
12, Sandan Osa-cho Matsugasaki Sakyo-ku
Kyoto-shi Kyoto(JP)

(74) Representative: von Kreisler, Alek, Dipl.-Chem. et al
Patentanwälte Von Kreisler-Selting-Werner
Deichmannhaus am Hauptbahnhof
D-5000 Köln 1(DE)

(54) **Cephalosporin compound, method of its production and composition for prevention and treatment of bacterial infectious diseases.**

(57) A cephalosporin compound represented by the formula (I):

EP 0 300 423 A2

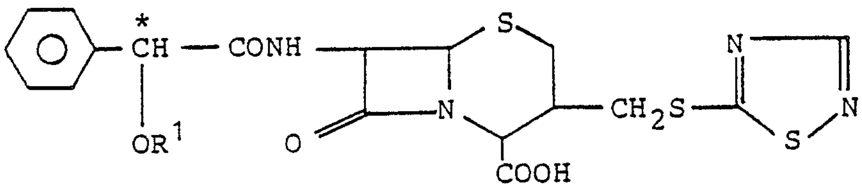 (I)

wherein $R^1$ represents a hydrogen atom or α-amino acid residue (ester bonding), its ester, or its pharmacologically acceptable salt, which is useful as a preventive, therapeutic drug for bacterial infections as well as an intermediate for the synthesis of other cephalosporin compounds, a method of its production, a pharmaceutical composition for prevention and treatment of bacterial infectious diseases and method for the treatment of bacterial infectious diseases.

# CEPHALOSPORIN COMPOUND, METHOD OF ITS PRODUCTION AND COMPOSITION FOR PREVENTION AND TREATMENT OF BACTERIAL INFECTIOUS DISEASES

## BACKGROUND OF THE INVENTION

### 1. Field of the Invention

The present invention relates to a new cephalosporin compound which is useful as a preventive, therapeutic drug for bacterial infections as well as an intermediate for the synthesis of other cephalosporin compounds, a method of its production, a pharmaceutical composition for prevention, treatment of bacterial infectious diseases and methods for the treatment of bacterial infectious diseases.

### 2. Description of the Prior Art

The present inventors previously developed a compound represented generally by the formula (A):

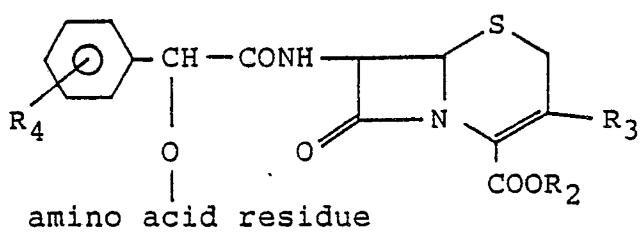

(A)

(WO84/01949).

In general, cephalosporin compounds are slightly absorbable into blood when administered orally, and therefore they are usually administered by injection. However, the cephalosporin compound (A), even when administered orally, is absorbed via digestive tract and hydrolyzed to be a cephalosporin compound represented generally by the formula (A-1):

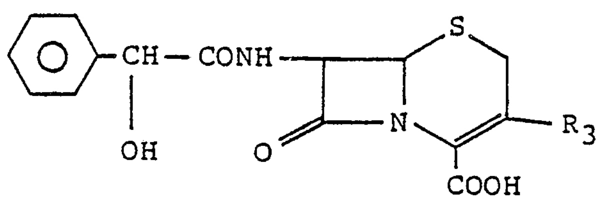

(A-1)

[hereinafter referred to as cephalosporin compound (A-1)], which exhibits excellent antibacterial activities. The cephalosporin compound (A) is a very excellent compound as stated above, but further technical progress will be achieved if a cephalosporin compound having more excellent characteristics is obtained.

An object of this invention is to provide a new cephalosporin compound possessing antibacterial activities against Gram-negative bacteria and noticeable antibacterial activities against Gram-positive bacteria, specifically to provide a new cephalosporin compound possessing noticeable antibacterial activities against sensitive staphylococci and resistant staphylococci.

Another object of this invention is to provide a cephalosporin compound which is easily absorbed into blood even when administered orally.

A still another object of this invention is to provide a production method of said cephalosporin compound.

A still another object of this invention is to provide a pharmaceutical composition containing said

cephalosporin compound which is for prevention and treatment of bacterial infectious diseases.

A still another object of the present invention is to provide methods for the treatment of bacterial infectious diseases by means of administration of said cephalosporin antibiotics.

## SUMMARY OF THE INVENTION

Taking note of the basic structure of the cephalosporin compounds (A) and (A-1), the present inventors made further investigations and found that only when $R^3$ in the formulae (A) and (A-1) is a 1,2,4-thiadiazol-5-yl-thiomethyl group, the antibacterial activities against Gram-positive bacteria further improves than the cephalosporin compounds (A) and (A-1), preserving strong antibacterial activities against Gram-negative bacteria, and that some of such compounds are absorbable into blood even when administered orally. Based on these findings, the inventors completed this invention.

Accordingly, the present invention relates to a cephalosporin compound represented by the formula (I):

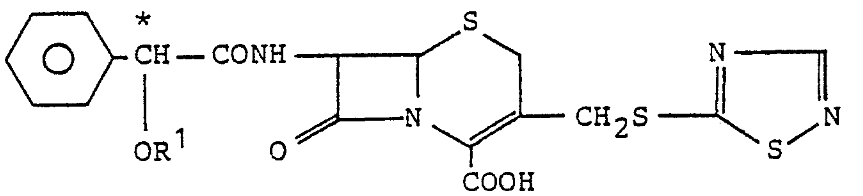

(I)

wherein $R^1$ represents a hydrogen atom or α-amino acid residue (ester bonding) [hereinafter also referred to as cephalosporin compound (I)], its ester, its pharmacologically acceptable salt, a method of their production, a pharmaceutical composition containing them, which is for prevention and treatment of bacterial infectious diseases and methods for the treatment of infectious diseases.

## DETAILED DESCRIPTION OF THE INVENTION

For $R^1$ in the formula (I), the amino acid residue is an α-amino acid residue cooperating with the adjacent oxygen atom to form an ester bonding. Said amino acid residue may be substituted at its amino group by a lower alkyl, preferably by a lower alkyl group having 1 to 4 carbon atoms, e.g. methyl, ethyl, propyl, isopropyl and butyl. Said amino acid residue may be in the form of any of D-configuration, L-configuration and DL-configuration. Examples of such amino acid residue include the following:

Neutral amino acid residues:

Aliphatic amino acid residues (glycyl, alanyl, valyl, leucyl, isoleucyl, etc.), oxy amino acid residues (seryl, threonyl, etc.), sulfur-containing amino acid residues (cysteinyl, cystinyl, methionyl, etc.), amide amino acid residues (asparaginyl, glutaminyl, etc.), aromatic amino acid residues (phenylalanyl, tyrosyl, tryptophyl, etc.).

Acidic amino acid residues:

Aspartyl, glutamyl, etc.

Basic amino acid residues:

Histidyl, lysyl, arginyl, etc.

Imino acids:

Prolyl, oxyprolyl, etc.

When the cephalosporin compound (I) is of the ester type, it is preferable that the ester be an ester in which the hydrogen atom of the carboxyl group in the formula (I) has been substituted by an alkyl group, phthalidyl, 5-methyl-1,3-dioxol-2-on-4-ylmethyl, or a group of the formula:

$$-\underset{R^2}{\underset{|}{C}}HOCOR^3$$

(G)

wherein $R^2$ represents an alkyl group and R3 represents an alkyl group or alkoxy group.

Examples of the alkyl group include lower alkyl groups having 1 to 4 carbon atoms, such as methyl, ethyl, n-propyl, isopropyl and butyl.

It is preferable that the group represented by the formula (G) be a 1-alkanoyloxyalkyl group or 1-alkoxycarbonyloxyalkyl group.

The alkyl group for R2 in the formula (G) may be straight or branched and examples include alkyl groups having 1 to 4 carbon atoms, such as methyl, ethyl, n-propyl, isopropyl, n-butyl and t-butyl.

The alkyl group or alkoxy group for $R^3$ in the formula (G) may be straight or branched, having 1 to 10 carbon atoms, preferably 1 to 7 carbon atoms.

Examples of the l-alkanoyloxyalkyl group in the formula (G) include acetoxymethyl, propionyloxymethyl, isopropionyloxymethyl, n-butyryloxymethyl, isobutyryloxymethyl, pivaloyl oxymethyl, n-valeryloxymethyl, 2-methylbutyryloxymethyl, isovaleryloxymethyl, n-hexanoyloxymethyl, 3-methylvaleryloxymethyl, neohexanoyloxymethyl, 2-methylhexanoyloxymethyl, 2,2-dimethylbutyryloxymethyl, diethylacetoxymethyl, dipropylacetoxymethyl, 2,2-dimethylvaleryloxymethyl, neoheptanoyloxymethyl, cyclohexanoyloxymethyl, cyclohexylacetoxymethyl,1-acetoxyethyl, 1-n-propionyloxyethyl, 1-n-butyryloxyethyl, 1-isobutyryloxyethyl, 1-n-valeryloxyethyl, 1-pivaloyloxyethyl, 1-isovaleryloxyethyl, 1-n-hexanoyloxyethyl, and 1-cyclohexanoyloxyethyl.

Examples of the 1-alkoxycarbonyloxyalkyl group in the formula (G) include 1-methoxycarbonyloxyethyl, 1-ethoxycarbonyloxyethyl, 1-n-propoxycarbonyloxyethyl, 1-isopropoxycarbonyloxyethyl, 1-n-butoxycarbonyloxyethyl, 1-tert-butoxycarbonyloxyethyl, 1-pentyloxycarbonyloxyethyl, and 1-hexylcarbonyloxyethyl.

Examples of particularly preferable cephalosporin compounds of the ester type include esters of acetoxymethyl, propionyloxymethyl, n-butyryloxymethyl, isovaleryloxymethyl, pivaloyloxymethyl, 1-acetoxyethyl, 1-propionyloxyethyl, 1-isobutyryloxyethyl, 1-n-valeryloxyethyl, 1-isovaleryloxyethyl, 1-pivaloyloxyethyl, phthalidyl, 1-ethoxycarbonyloxyethyl, 5-methyl-1,3,-dioxol-2-on-4-ylmethyl, etc.

The cephalosporin compound (I) forms at its amino acid residue a pharmacologically acceptable salt, preferably an acid addition salt. There is no particular limitation to the choice of the acid to form the acid addition salt as long as it is capable of forming a salt with the amino acid residue moiety and pharmacologically acceptable. Examples of such acids include mineral acids such as hydrochloric acid, sulfuric acid, phosphoric acid and nitric acid; and organic acids such as oxalic acid, fumaric acid, maleic acid, citric acid, tartaric acid, methanesulfonic acid and toluenesulfonic acid.

The cephalosporin compound (I) of this invention may also form a salt at its carboxyl group. Examples of the salt include alkali metal salts (e.g. sodium salt, potassium salt), alkaline earth metal salts (e.g. calcium salt) and organic base salts (e.g. pyridine salt).

It is preferable that the cephalosporin compound (I) be in the form of a D-configuration with respect to the carbon atom marked with asterisk (*) in the formula (I).

The cephalosporin compound (I), its ester and its pharmacologically acceptable salt can be produced by the methods 1 through 4 described below, for instance.

Method 1:

The method in which reaction is carried out between a compound represented by the formula (II):

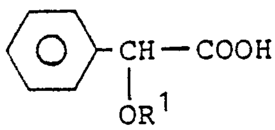

(II)

wherein $R^1$ has the same definition as above [hereinafter referred to as compound (II)] and a compound represented by the formula (III):

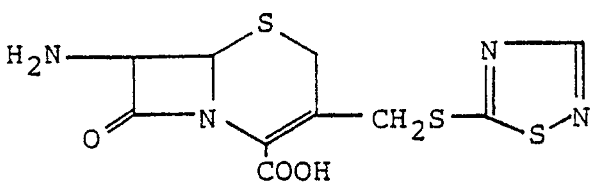

(III)

[hereinafter referred to as compound (III)], its ester or its salt.

The compound (II) is used in the reaction directly in the form of a free carboxylic acid or after being converted to a reactive derivative thereof; these modes are both involved in this invention. Accordingly, the compound (II) is used in the acylation reaction in the form of a free acid or after being converted to a salt of sodium, potassium, calcium, triethylamine, pyridine, etc., or to a reactive derivative thereof such as an acid halide (acid chloride, acid bromide, etc.), an acid anhydride, a mixed acid anhydride [substituted phosphoric acid, dialkylphosphoric acid, etc.), alkylcarbonic acid (monoethylcarbonic acid, etc.), etc.], an active amide (amide with imidazole, etc.) or an ester (cyanomethyl ester, 4-nitrophenyl ester, etc.), or further to a compound represented by the formula (II-1):

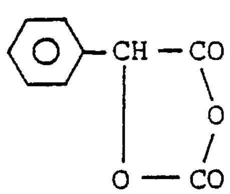

(II-1)

[hereinafter referred to as compound (II-1)].

When the compound (II) is used in the form of a free acid or salt, it is preferable that this reaction be carried out in the presence of a condensing agents. Examples of the condensing agent include dehydrating agents such as N-N-disubstituted carbodiimides (e.g. N,N-dicyclohexylcarbodiimide), carbodiimide compounds (e.g. 1-ethyl-3-(3′-dimethylaminopropyl)carbodiimide, N-cyclohexyl-N′-morpholinoethylcarbodiimide, N-cyclohexyl-N′-(4-diethylaminocyclohexyl)carbodiimide) and azolide compounds (e.g. N-N-carbonyldiimidazole, N,N-thionyldiimidazole); and reagents (so-called Vilsmeier reagents) resulting from reaction between an amide compound such as N-methylformamide or N,N-dimethylformamide and a halide such as thionyl chloride, phosphorus oxychloride or phosgene. When these condensing agents are used, the reaction is thought to proceed via reactive derivative of the carboxylic acid.

When the amino group of the amino acid residue represented by $R^1$ in the compound (II) in the present reaction is a primary or secondary amino group, it is preferable that the amino group be protected by an amino-protective group such as 2,2,2-trichloroethoxycarbonyl group, 2-methylsulfonylethyloxycarbonyl group, t-butoxycarbonyl group (hereinafter also referred to as BOC), chloroacetyl group or trityl group.

The reaction between the compound (II) and the compound (III) is normally carried out in a solvent inert to the reaction; such solvents include polar solvents such as dichloromethane, chloroform, acetone, tetrahydrofuran, dioxane, acetonitrile, methyl isobutyl ketone, dimethylformamide, dimethylsulfoxide, hexamethylphosphoric triamide and sulfolane; non-polar solvents such as benzene, toluene, petroleum ether and n-hexane; and mixed solvents thereof. As the case may be, aqueous mixed solvents thereof can also

be used.

Reaction temperature for the reaction between the compound (II) and the compound (III) can be arbitrarily chosen, but it is usually below 50 °C.

Particularly when $R_1$ is an α-amino acid residue, the reaction is carried out at room temperature or under cooling conditions (-20 to 0 °C).

The compounds (II) and (II-1) are generally known; the compound (II-1) can be produced by the method described in the official gazettes of Japanese Patent Publication Nos. 45880/1974 and 37997/1980, for instance, or by a method in accordance with them.

The compound (III) in a free form or its pharmacologically acceptable salt can, for example, be produced by the well-known method in which reaction is carried out between 7-aminocephalosporanic acid and 1,2,4-thiadiazol-5-thiol (described in the specification of U.S. patent No. 3,979,383 and the official gazettes of Japanese Patent Application laid open Nos. 90907/1978, 904/1980, 49303/1980 and 82392/1982, for instance).

The compounds (III) of the ester type are new compounds and they are produced by reaction between a compound represented by the formula (IV):

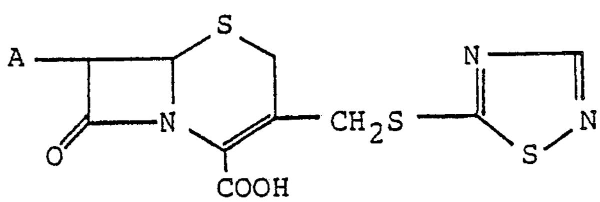

(IV)

wherein A represents an amino group or protected amino group [hereinafter referred to as compound (IV)] and a compound represented by the formula (V):

$X—R^4$ (V)

wherein R represents a group to be introduced by the esterification; and X represents a group reactive with carboxyl group, such as halogen, alkylsulfonate or arylsulfonate. Note that for producing an ester of the type:

— $CHOCOR^3$
  |
  $R^2$
(G)

wherein $R^2$ and $R^3$ have the same definitions as above, reaction is carried out between the compound (IV) and a compound represented by the formula (V-1):

$R^3OCOCH$ —X
  |
  $R^2$
(V-1)

wherein $R^2$, $R^3$ and X have the same definitions as above [hereinafter referred to as compound (V-1)] to give the desired compound (III) of the ester type; when A is a protected amino group, the compound (III) of the above-mentioned ester type can be obtained by deprotecting with a standard method the ester thus obtained.

As regards X in the formula (V), preferred halogens include chloro, bromo and iodo, and the preferred alkyls for the alkylsulfonate are alkyls having 1 to 4 carbon atoms such as methyl, ethyl, propyl, isopropyl and butyl. Specific examples of such alkylsulfonates include methanesulfonate and ethanesulfonate. The preferred aryl for the arylsulfonate is phenyl; specific examples of such arylsulfonates include benzenesulfonate and toluenesulfonate.

The compound (IV) may be used directly in the reaction, but it is convenient that it be converted to a reactive derivative before being reacted. The reactive derivative of the compound (IV) is a reactive derivative with respect to its carboxy group; examples include reactive derivatives generally used for esterification. Usable reactive derivatives also include inorganic salts such as alkali metal salts (sodium salt, potassium salt, etc.) and alkaline earth metal salts (calcium salt, magnesium salt, etc.); and organic salts such as organic amine salts (triethylamine, pyridine, etc.).

This reaction is preferably carried out at room temperature or, as necessary, under cooling conditions to

prevent by-production of $\Delta^2$-isomers.

The reaction can be easilly proceeded in the presence of a solvent which does not take part in the reaction, such as dimethylformamide, dimethylacetamide, hexamethylene phosphoric triamide, acetone or acetonitrile.

In the present reaction, it is preferable that A in the formula (IV) be a protected amino group; in this case, reaction between the compound (IV) and the compound (V) gives a compound of the formula (III) wherein the 7-position amino group is protected, and the protective group can be eliminated by a well known method of protective group elimination.

When A in the above general formula (IV) is a protected amino group, usable protective groups for the amino group include protective groups which are well known in the field of cephalosporin compounds. As the protective group for the amino group, a well-known amino-protective group is selected as appropriate, for example, furoyl, formyl, monochloroacetyl, dichloroacetyl, trichloroacetyl, trifluoroacetyl, methoxycarbonyl, ethoxycarbonyl, t-butoxycarbonyl, 2,2,2-trichloroethoxycarbonyl, benzyloxycarbonyl, p-nitrobenzyloxycarbonyl, diphenylmethyloxycarbonyl, methoxymethyloxycarbonyl, trimethylsilyl, benzylcarbonyl, 2-thienylacetyl, 5-amino-5-carboxyvaleryl, carbobenzyloxy, trityl, phthalimide and orthohydroxybenzylidene group.

Specific examples of methods of eliminating the protective group include the method in which the protective group is decomposed with methanol after iminochlorination with phosphorus pentachloride for elimination of benzylcarbonyl, 2-thienylacetyl, 2-furylacetyl, D-5-amino-5-carboxyvaleryl, etc.; the method using acid (e.g. formic acid, trifluoroacetic acid) in the elimination of trityl, orthohydroxybenzylidene, etc., and the Ingemans method using hydrazine for elimination of phthalimide, and the like.

Method 2:

The method in which a compound represented by the formula (VI):

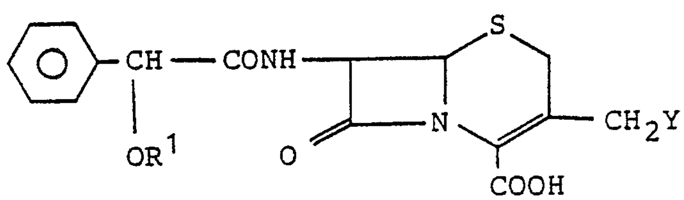

(VI)

wherein $R^1$ has the same definition as above; Y represents a nucleophilic residue [hereinafter referred to as compound (VI)], its ester or its salt, after nucleophilic substitution of its 3-position Y with a 1,2,4-thiadiazol-5-thiol, is esterified or deesterified as necessary, and further converted to a pharmacologically acceptable salt or a free acid as necessary.

Examples of the above-mentioned nucleophilic group include acetoxymethyl group and halogens (iodide, bromo, etc.).

To the nucleophilic substitution of the 3-position Y of the compound (VI) with 1,2,4 thiadiazol-5-thiol, a well-known method is applicable [described in "Cephalosporins and Penicillins, Chemistry and Biology", edited by E. Flynn, Academic Press, 1972, Chap. 4, p.158; the specification of Japanese Patent Publication No. 31356/1977; "Recent Advances in the Chemistry of $\beta$-Lactam Antibiotics", Cambridge, England, 28-30, June, 1976 (The Chemical Society, Burlington House, London W1V OBN), p. 109; Tetrahedron Letters, 22, 3915 (1981)].

The compound (VI) is produced as follows:

That is, the compound (VI) can be produced by treating the compound (II) and a compound represented by the formula (VII):

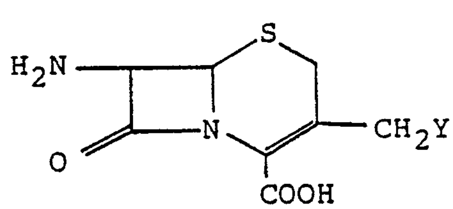

(VII)

wherein Y has the same definition as above [hereinafter referred to as compound (VII)], its ester or its salt in the same manner as in the production method for the compound (I) in Method 1 described above. Compounds represented by the formula (VII) and having a halogen atom for Y can also be produced by the method described in "Recent Advances in the Chemistry of β-Lactam Antibiotics", edited by J. Elks, Cambridge, England, 28-30, June, 1976 (the Chemical Society, Burlington House, London WIV OBN), p. 106, and Tetrahedron Letters, 22, 3915 (1981), for instance.

Method 3:

The method in which reaction is carried out between a compound represented by the formula (I-1):

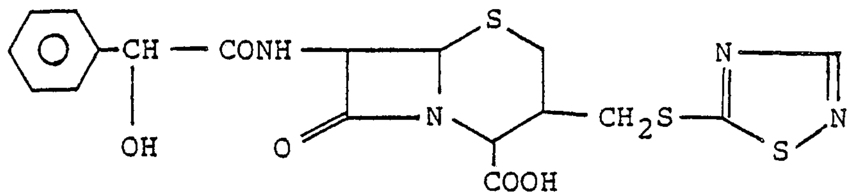

(I-1)

[hereinafter referred to as compound (I-1)], ( its ester or its salt and a compound represented by the formula (VIII):

$R^5$—OH (VIII)

wherein $R^5$ represents an amino acid residue [hereinafter referred to as compound (VIII)].

The compound (VIII) is used in the reaction directly in the form of a free carboxylic acid or after being converted to a reactive derivative thereof; these modes are both involved in this invention. Accordingly, the compound (VIII) is used in the acylation reaction in the form of a free acid or after being converted to a salt of sodium, potassium, calcium, triethylamine, pyridine, etc., or to a reactive derivative thereof such as an acid halide (acid chloride, acid bromide, etc.), an acid anhydride, a mixed acid anhydride [substituted phosphoric acid (dialkylphosphoric acid, etc.), alkylcarbonic acid (monoethylcarbonic acid, etc.), etc.], an active amide (amide with imidazole, etc.) or an ester (cyanomethyl ester, 4-nitrophenyl ester, etc.).

When the compound (VIII) is used in the form of a free acid or salt, it is preferable that an appropriate condensing agent be used. Examples of usable condensing agents include dehydrating agents such as N,N-di-substituted carbodiimides (e.g. N,N-dicyclohexylcarbodiimide), carbodiimide compounds (e.g. 1-ethyl-3-(3′-dimethylaminopropyl)carbodiimide, N-cyclohexyl-N′-morpholinoethylcarbodiimide, N-cyclohexyl-N′-(4-diethylaminocyclohexyl)carbodiimide) and azolide compounds (e.g. N,N-carbonyldiimidazole, N,N-thionyldiimidazole). When these condensing agents are used, the reaction is thought to proceed via reactive derivative of the carboxylic acid. It is preferable that a base such as 4-dimethylaminopyridine be used as the catalyst for the reaction.

When the amino group represented by $R^5$ in the compound (VIII) in the present reaction is a primary or secondary amino group, it is preferable that this amino group be protected with a protective group such as 2,2,2-trichloroethoxycarbonyl group, 2-methylsulfonylethyloxycarbonyl group, t-butoxycarbonyl group, chloroacetyl group or trityl group.

The reaction is normally carried out in an inert solvent. Examples of the solvent include organic solvents such as water, acetone, dioxane, acetonitrile, chloroform, benzene, methylene chloride, ethylene chloride, tetrahydrofuran, ethyl acetate, N,N-dimethylformamide and pyridine, and mixtures thereof.

This reaction is preferably carried out under cooling conditions to prevent by-production of $\Delta^2$-isomers, and the reaction can be easily proceeded in the presence of a solvent which does not interfere with the reaction (e.g. dimethylformamide, dimethylacetamide, dimethylsulfoxide, hexamethylene phosphoric triamide, acetone, acetonitrile).

Method 4:

The method in which reaction is carried out between a compound represented by the formula (I-2):

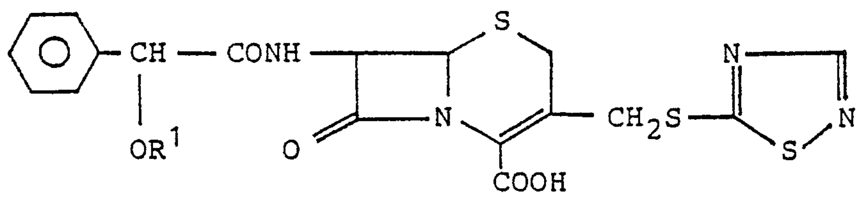

(I-2)

wherein $R^1$ has the same definition as above [hereinafter referred to as compound (I-2)] and the compound (V).

By this method, a cephalosporin compound of an ester type is produced from the cephalosporin compound (I-2) in the form of a free acid.

As regards this reaction, it is preferable that the compound (I-2) be used in the reaction after being converted to a reactive derivative thereof (e.g. alkali metal salt such as sodium salt or potassium salt; alkaline earth metal salt such as calcium salt; triethylamine salt; or pyridine salt); these modes are also involved in this invention.

This reaction is preferably carried out under cooling conditions to prevent by-production of $\Delta^2$-isomers, and the reaction can be easily proceeded in the presence of a solvent which does not interfere with the reaction (e.g. dimethylformamide, dimethylacetamide, hexamethylene phosphoric triamide, acetone, acetonitrile).

When the amino group for $R^1$ in the formula (I-2) in the present reaction is a primary or secondary amino group, it is preferable that the amino group be protected; in this case, reaction between the compound (I-2) and the compound (V) gives the cephalosporin compound (I) in the form of a free acid in which the amino group has been protected, and the protective group can be eliminated by a well-known method of protective group elimination.

The cephalosporin compound (I) can be converted to a pharmacologically acceptable salt thereof by a well-known method.

The cephalosporin compound (I), its ester and its pharmacologically acceptable salt can be separated and purified in accordance with a standard method.

The cephalosporin compound (I) thus obtained, its ester, and its pharmacologically acceptable salt are separated from the reaction mixture by a standard method. For example, they can be purified by absorption to an absorptive resin such as Amberlite XAD-2 (Rohm and Haas Co.) or Diaion HP-20 (Mitsubishi Chemical Industries Co.) followed by elution with an aqueous organic solvent. Chromatography with Sephadex LH-20, G-10 (Pharmacia Co.) etc., if necessary, is also effective.

The cephalosporin compound (I) of the present invention and its pharmacologically acceptable salt possess very excellent antibacterial activities against Gram-positive bacteria, specifically sensitive staphylococci and resistant staphylococci, including Staphylococcus aureus and Staphylococcus epidermidis, as well as against Gram-negative bacteria such as Escherichia coli, Klebsiella pneumoniae, Proteus vulgaris, Proteus mirabilis and Proteus morganii. Accordingly, the cephalosporin compound (I) of this invention and its pharmacologically acceptable salt are useful antibacterials having noticeably improved antibacterial activities against Gram-positive bacteria as well as antibacterial activities against Gram-negative bacteria. Moreover, these compounds are extremely low in toxicity.

In addition, when orally administered, the cephalosporin compound (I) of the above-mentioned easily decomposable ester type, specifically of the easily hydrolyzable ester type, in particular of the ester type having a group represented by the formula:

$$-\underset{\substack{|\\ R^2}}{C}HOCOR^3$$

(G)

wherein R2 and R3 have the same definitions as above, a phthalidyl group, a 5-methyl-1,3-dioxol-2-on-4-ylmethyl group or the like, is rapidly absorbed into blood to give a high blood concentration of a cephalosporin compound represented by the formula (I), and this high blood concentration is maintained for a long time. Furthermore, when the cephalosporin compound (I) of said easily decomposable ester type is converted to its acid addition salt, its solubility in the digestive tract noticeably increases to further improve absorption efficiency, i.e., the absorbability of the cephalosporin compound (I) into blood further increases.

Furthermore, the compound (I) which has an amino acid residue for $R^1$ and which is of the easily biohydrolyzable ester type is very useful as an oral prodrug of the compound (I) having a hydrogen atom for $R^1$.

Therefore, the cephalosporin compound (I) of this invention, its ester and its pharmacologically acceptable salt is very useful as preventive, therapeutic drugs for bacterial infectious diseases. Said preventive, therapeutic drugs for bacterial infectious diseases can be used, for example, as preventive, therapeutic drugs for bacterial diseases (e.g. suppurative diseases, respiratory infections, biliary tract infections, urinary tract infections) in warm-blooded animals including humans (e.g. dogs, cat, bovines, horses, rats, mice).

The cephalosporin compound (I) according to this invention, its ester or its pharmacologically acceptable salt can be used as a preventive, therapeutic drug for infectious diseases singly or in a form of pharmaceutical composition. The preventive, therapeutic drug for bacterial infections of the compound (I) of this invention is prepared as an appropriate dosage form (e.g. oral preparation such as tablets, sugar-coated tablets, capsules, fine subtilaes, granules or dry syrup; or parenteral preparation such as injection, suppositories or external preparation) by mixing together a therapeutically effective amount of the compound (I) and a pharmacologically acceptable excipient or additive (e.g. diluent, filler, emulsifier, lubricant, flavor, cologing agent or other additive) as necessary. The compound (I) in the form of a salt is soluble in water; thus it can be prepared as an injection, for instance.

The preventive, therapeutic drug for infectious diseases of the present invention can be produced by dilution with a pharmaceutical excipient according to a well-known method. For example, as regards an orally administered therapeutic drug for bacterial infectious diseases, excipients such as starch, lactose, sugar, calcium carbonate, calcium phosphate, etc. are used.

Meanwhile, it is preferable that an organic acid be further added to said orally administered therapeutic drug for bacterial infectious diseases; thus the solubility of the cephalosporin compound (I) in the digestive tract increases to facilitate its absorption into blood. There is no particular limitation to the choice of the organic acid as long as it is pharmacologically acceptable; preferred examples include organic carboxylic acids such as maleic acid, fumaric acid, tartaric acid, citric acid, succinic acid, malic acid, oxalic acid, mandelic acid, malonic acid and benzoic acid. The amount of the added organic acid is usually 0.01 to 20 moles, preferably 0.02 to 2 moles to 1 mole of the cephalosporin compound (I), its ester or its salt.

Other additives may be further added to said orally administered therapeutic drug for bacterial infectious diseases, if desired. Examples of preferred additives to be further added include binders (e.g. starch, gum arabic, carboxymethylcellulose, hydroxypropylcellulose, crystalline cellulose), lubricants (e.g. magnesium stearate, talc) and disintergrators (e.g. carboxymethylcellulose calcium, talc).

The cephalosporin compound (I) of this invention, its ester or its pharmacologically acceptable salt may be used in combination with other antibacterially active substances; for example, antibacterials (penicillins, aminoglucosides, cephalosporins, etc.) or therapeutic drugs for systemic symptoms due to bacterial infection (antipyretics, analgesios, anti-inflammatory drugs, etc.).

The preventive, therapeutic drug for bacterial infectious diseases comprising the cephalosporin compound (I) of the above-mentioned easily decomposable ester type of this invention, specifically the cephalosporin compound (I) of the ester type having an $\alpha$-amino acid residue for $R^1$, when orally administered, is rapidly absorbed via digestive tract and immediately hydrolyzed by enzymes in the living body to cause elimination of $R^1$ from the cephalosporin compound (I) along with conversion to a deesterified active ingredient compound or its pharmacologically acceptable salt, which in turn exhibits its excellent antibacterial activities.

The dosage amount of the cephalosporin compound (I), its ester and its pharmacologically acceptable salt varies depending upon the administration subject, symptoms, and other factors; for example, when administered to an adult with supporative disease, they are orally administered at a dose level of about 1 to 40 mg/kg b.w. and 1 to 4 times daily, as calculated on the basis of the hydrolyzed compound corresponding to the cephalosporin compound (I).

Antibacterial activity test

The minimum inhibitory concentration (MIC,$\mu$g/ml) of the compound of the present invention was determined using cefaclor, which is said to be particularly effective on staphylococci, streptococci, pneumococci, etc., as the control substance. The results are shown in Table 1.

Table 1

| | Staphylococcus aureus Smith | Escherichia coli NIH |
|---|---|---|
| Compound of Example 1 | 0.2 | 0.39 |
| Cefaclor | 1.56 | 0.78 |

Acute toxicity test

The acute toxicity of the orally administered cephalosporin derivative of this invention in mice was determined. The test is summarized as follows:

Subject animals: Male mice (ICR, at 5 weeks in age), n = 3

Method of administration: The cephalosporin derivative as obtained in Example 3 below was dissolved in distilled water, and this aqueous solution was orally administered.

Result: The $LD_{50}$ value of the compound of Example 3 was less than 5.0 g/kg.

Oral administration experiment

The urinary recovery rate of the orally administered hydrolyzed compound of the cephalosporin derivative of this invention to humans was determined. The experiment is summarized as follows:

Control compound: Pivaloyloxymethyl 7-[D-O-(L-alanyl)mandelamido]-3-[(5-methyl-1,3,4-thiadiazol-2-yl)-thiomethyl]-3-cephem-4-carboxylate hydrochloride

Method of administration: A 125-mg capsule was orally administered as the hydrolyzed compound.

Method of determination: Bacillus subtiliss was used for the determination.

Results

| Compound | Urinary recovery rate (%), 0-8 hours |
|---|---|
| Compound of Example 3 | 56.0 |
| Control compound | 38.0 |

Example 1

7-D-(-)Mandelamido-3-(1,2,4-thiadiazol-5-yl)thiomethyl-3-cephem-4-carboxylic acid

1.07 g of sodium bicarbonate was dissolved in 22.5 ml of water. To this solution, 1.2 g of 7-amino-3-(1,2,4-thiadiazol-5-yl)thiomethyl-3-cephem-4-carboxylic acid and 20 ml of acetone were added. While stirring the resulting solution, solution of 0.94 g of D-(-)-O-formylmandelic acid chloride in 2 ml of acetone was added dropwise at -5°C over a period of 20 minutes. After two hours of agitation at the same temperature, the acetone was distilled off under reduced pressure. 22.5 ml of water and 2.6 g of sodium bicarbonate were then added, and this was followed by 20 hours of agitation at room temperature. Hydrochloric acid was then added to adjust the mixture to pH 1, the separating substance was collected by

filtration, washed with water and dried to thereby give 1.5 g of the subject compound.

I R(KBr, $cm^{-1}$); 3400, 1780, 1680, 1520

N M R ($DMSO_{d\text{-}6}$, $\delta$ ppm); 3.5~5.0 (br, 1H, $-CO_2H$), 3.64 (brs, 2H, 2-position $-H_2$), 4.28, 4.61 (ABq, J=13Hz, 2H, 3-position $-CH_2-$), 4.9~5.2 (m, 2H, 6-position -H, -CH<), 5.68 (d×d, J=5Hz, 8Hz, 1H, 7-position -H), 5.7~6.4 (br, 1H, -OH), 7.05~7.60 (m, 5H, phenyl), 8.64 (d, J=8Hz, 1H, -CONH-), 8.67 (s, 1H, thiadiazole 3-position -H).

Example 2

Pivaloyloxymethyl 7-D-(-)mandelamido-3-(1,2,4-thiadiazol-5-yl)thiomethyl-3-cephem-4-carboxylate

To 10 ml of dimethylformamide, 1 g of the compound of Example 1 and 254 mg of potassium acetate were added. To this mixture, 625 mg of iodomethyl pivalate was added at -15° C in a nitrogen gas flow, and this was followed by 2 hours of agitation at the same temperature. The reaction liquid was added to 50 ml of cold ethyl acetate and washed with sequential additions of cold water, aqueous sodium bicarbonate and aqueous sodium chloride. After drying with anhydrous Glauber's salt, the solvent was distilled off under reduced pressure. The residue was subjected to silica gel column chromatography and eluted with benzene-ethyl acetate, and the desired fraction was concentrated and crystallized from petroleum ether to thereby give 370 mg of the subject compound.

I R (Nujol, $cm^{-1}$); 1780, 1740, 1730, 1680

N M R ($CDCl_3$, $\delta$ ppm); 1.22 (s, 9H, $-C(CH_3)_3$), 2.2 (br, 1H, -OH), 3.62 (brs, 2H, 2-position $-H_2$), 4.16, 4.68 (ABq , J=13Hz, 2H, 3-position $-CH_2-$), 4.96 (d, J=5Hz, 1H, 6-position -H,), 5.13 (d, J=4Hz, 1H, >C$\underline{H}$-OH), 5.73 (d×d, J=5Hz, 9Hz, 1H, 7-position -H), 5.89 (s, 2H, $-OCH_2O-$), 7.3 (m, 6H, phenyl, -CONH-), 8.39 (s, 1H, thiadiazole 3-position -H).

The following compounds can be produced in the same manner as in Example 1 or 2 described above.

* 1-Acetoxyethyl 7-D-(-)mandelamido-3-(1,2,4-thiadiazol-5-yl)thiomethyl-3-cephem-4-carboxylate
* 1-Ethoxycarbonyloxyethyl 7-D-(-)mandelamido-3-(1,2,4-thiadiazol-5-yl)thiomethyl-3-cephem-4-carboxylate
* 1-Isopropoxycarbonyloxyethyl 7-D-(-)mandelamido-3-(1,2,4-thiadiazol-5-yl)thiomethyl-3-cephem-4-carboxylate
* (5-Methyl-1,3-dioxol-2-on-4-yl)methyl 7-D-(-)mandelamido3-(1,2,4-thiadiazol-5-yl)thiomethyl-3-cephem-4-carboxylate

Example 3

Pivaloyloxymethyl 7-[D-O-(L-alanyl)mandelamido]-3-[(1,2,4-thiadiazol-5-yl)thiomethyl]-3-cephem-4-carboxylate hydrochloride

(1) To 37 ml of water, 4.54 g of sodium bicarbonate was added, and 8.92 g of 7-amino-3-[(1,2,4-thiadiazol-5-yl)thiomethyl]-3-cephem-4-carboxylic acid was further added. They were dissolved while removing the carbon dioxide. To this solution, 37 ml of methanol and 4.23 ml of salicylaldehyde were added at room temperature, and this was followed by 20 minutes of agitation. After the addition of 110 ml of isopropanol, agitation was further conducted for 2 hours and 30 minutes, and the separating crystal was collected by filtration and dried to thereby give 10.15 g of sodium 7-[(O-hydroxybenzyliden)imino]-3-[(1,2,4-thiadiazol-5-yl)thiomethyl]-3-cephem-4-carboxylate.

I R (Nujol, $cm^{-1}$): 3400, 1765, 1625

N M R ($DMSO_{d\text{-}6}$, $\delta$ ppm): 3.0~4.2 (m, 2H, 2-position $-H_2$) 4.42, 4.72 (ABq, J=13Hz, 2H, 3-position $-CH_2-$), 5.20 (d, J=5Hz, 1H, 6-position -H), 5.48 (d, J=5Hz, 1H, 7-position -H), 6.6~7.8 (m, 4H, phenyl), 8.67 (s, 1H, thiadiazole 3-position -H), 8.74 (s, 1H, -CH=), 12.0 ~12.6 (br, 1H, -OH).

(2) To 125 ml of dimethylformamide, 5 g of the compound obtained in (1) was added. While stirring the mixture, 25 ml of solution of 3.98 g of iodomethyl pivalate in dimethylformamide was added at -15° C, and this was followed by 1 hour of agitation. The reaction liquid was extracted with 600 ml of 5% aqueous sodium chloride and 1 l of ethyl acetate, and the organic layer was washed with sequential additions of

water, aqueous sodium bicarbonate and aquous sodium chloride. After drying with anhydrous Glauber's salt, the organic layer was concentrated under reduced pressure and crystallized from petroleum ether to thereby give 3.1 g of pivaloyloxymethyl 7-[(O-hydroxybenzyliden)imino]-3-[(1,2,4-thiadiazol-5-yl)-thiomethyl]-3 cephem-4-carboxylate.

I R (Nujol, $cm^{-1}$): 1765, 1740, 1620, 1580

N M R ($CDCl_3$, $\delta$ ppm): 1.23 (s, 9H, $-C(CH_3)_3$, 3.51, 3.84 (ABq, J=17Hz, 2H, 2-position $-H_2$), 4.17, 4.75 (ABq, J=13Hz, 2H, 3-position $-CH_2-$), 5.10 (d, J=5Hz, 1H, 6-position -H), 5.35 (d, J=5Hz, 1H, 7-position -H) 5.77~6.07 (m, 2H, $-CH_2O-$), 6.7~7.6 (m, 4H, phenyl), 8.41 (s, 1H, thiadiazole 3-position -H), 8.57 (s, 1H, -CH=), 11.4~12.2 (br, 1H, -OH).

(3) 1.99 g of the compound obtained in (2) was dissolved in 28 ml of ethyl acetate. To this solution, 2.8 ml of methanol and 0.91 ml of concentrated hydrochloric acid were added, and this was followed by 30 minutes of agitation. To this mixture, 56 ml of ethyl acetate was added dropwise, and this was followed by 1 hour of agitation at 5° C. The separating crystal was collected by filtration and dried to thereby give 1.25 g of pivaloyloxymethyl 7-amino-3-[(1,2,4-thiadiazol-5-yl)thiomethyl]-3-cephem-4-carboxylate hydrochloride.

I R (Nujol, $cm^{-1}$): 1760, 1625, 1595

N M R ($DMSO_{d-6}$, $\delta$ ppm): 1.12 (s, 9H, $-C(CH_3)_3$), 3.82 (brs, 2H, 2-position $-H_2$), 4.30, 4.70 (ABq, J=13Hz, 2H, 3-position $-CH_2-$), 4.92~5.44 (m, 2H, 6-position -H, 7-position -H), 5.66~6.26 (m, 2H, $-CH_2O-$), 7.8 ~9.8 (br, 3H, $-NH_3^+$), 8.68 (s, 1H, thiadiazole 3-position -H)

(4) 1.19 g of the compound obtained in (3) was dissolved in 50 ml of methylene chloride, washed with aqueous sodium bicarbonate and aqueous sodium chloride, and dried with anhydrous Glauber's salt. The solvent was distilled off under reduced pressure, and the residue was solidified from mixture of isopropyl ether and petroleum ether to thereby give 1.05 g of pivaloyloxymethyl 7-amino-3-[(1,2,4-thiadiazol-5-yl)-thiomethyl]-3-cephem-4-carboxylate.

N M R ($CDCl_3$, $\delta$ ppm): 1.23 (s, 9H, $-C(CH_3)_3$), 3.2~4.2 (br, 2H, $-NH_2$), 3.51, 3.82 (ABq, J=17Hz, 2H, 2-position $-H_2$), 4.16, 4.70 (ABq, J=13Hz, 2H, 3-position $-CH_2-$), 4.65~5.03 (m, 2H, 6-position -H, 7-position -H), 5.77~6.03 (m, 2H, $-CH_2O-$), 8.41 (s, 1H, thiadiazole 3-position -H).

(5) To 19 ml of methylene chloride, 950 mg of the compound obtained in (4) and 804 mg of D-O-(BOC-L-alanyl)mandelic acid were added. To this mixture, 473 mg of 1-ethyl-3-(3′-dimethylaminopropyl)-carbodiimide hydrochloride was added at -10° C, and this was followed by 30 minutes of agitation at the same temperature. 200 ml of methylene chloride was then added, and the mixture was washed with 10% aqueous solution of citric acid and aqueous sodium chloride, and dried with anhydrous Glauber's salt. After concentration under reduced pressure, the residue was solidified with isopropyl ether and subjected to silica gel column chromatography (benzene-ethyl acetate = 5:1), and the desired fraction was concentrated and solidified with petroleum ether to thereby give 925 mg of pivaloyloxymethyl 7-[D-O-(BOC-L-alanyl)-mandelamidol-3-[(1,2,4-thiadiazol-5-yl)thiomethyl]-3-cephem-4-carboxylate.

I R (Nujol, $cm^{-1}$): 1790, 1750, 1690

N M R ($CDCl_3$, $\delta$ ppm): 1.23 (s, 9H, $-C(CH_3)_3$), 1.40 (s, 9H, -BOC), 1.47 (d, J=7Hz, 3H, $-CH_3$), 3.62 (brs, 2H, 2-position $-H_2$), 4.0~4.5 (m, 1H, -CH<), 4.16, 4.70 (ABq , J=13Hz, 2H, 3-position $-CH_2-$), 4.96 (d, J=5Hz, 1H, 6-position -H), 5.70 (d×d, J=5Hz, 8Hz, 1H, 7-position -H), 5.90 (s, 2H, $-CH_2O-$), 6.15 (s, 1H, -CH<) 7.15~7.75 (m, 7H, -CONH-, -NH-, phenyl), 8.41 (s, 1H, thiadiazole 3-position -H).

(6) 800 mg of the compound obtained in (5) was dissolved in 4 ml of formic acid. To this solution, 0.5 ml of solution of 10.7 N hydrogen chloride in ethanol was added at 5° C, and this was followed by 10 minutes of agitation. This mixture was then poured into isopropyl ether, and the separating solid was collected by filtration and dissolved in methanol. The resulting solution was poured into isopropyl ether to give 625 mg of the subject compound.

I R (Nujol, $cm^{-1}$): 1790, 1775, 1690, 1625

N M R ($DMSO_{d-6}$, $\delta$ ppm): 1.16 (s, 9H, $-C(CH_3)_3$), 1.47 (d, J=7Hz, 3H, $-CH_3$), 3.65 (brs, 2H, 2-position $-H_2$), 4.0~4.5 (m, 1H, -CH<), 4.23, 4.64 (ABq, J=13Hz, 2H, 3-position $-CH_2-$), 5.06 (d, J=5Hz, 1H, 6-position -H), 5.70 (d×d, J=5Hz, 8Hz, 1H, 7-position -H), 5.90 (brs, 2H, $-CH_2O-$), 6.10 (s, 1H, -CH<), 7.20~7.7 (m, 5H, phenyl), 8.4~9.0 (m, 3H, $-NH_3^+$), 8.67 (s, 1H, thiadiazole 3-position -H), 9.44 (d. J=8Hz, 1H, -CONH-)

Example 4

240 mg of the compound of Example 3 (4) was dissolved in 5 ml of methylene chloride. To this solution, 0.2 ml of dimethylformamide was added, and the solution was cooled to -5° C. While stirring the solution, 180 mg of D-O-(L-alanyl)mandelic acid chloride hydrochloride was added, and this was followed by 1 hour of agitation. The methylene chloride was then distilled off under reduced pressure, and the residue

was solidified with isopropyl ether to thereby give 336 mg of pivaloyloxymethyl 7-[D-O-(L-alanyl)-mandelamido]-3-[(1,2,4-thiadiazol-5-yl)thiomethyl]-3-cephem-4-carboxylate hydrochloride.

The physical data of the compound obtained here were the same as those of the subject compound of Example 3.

Example 5

(1) 370 mg of pivaloyloxymethyl 7-D-mandelamido-3-(1,2,4-thiadiazol-5-yl)thiomethyl-3-cephem-4-carboxylate was dissolved in 3.7 ml of dry methylene chloride. To this solution, 176 mg of BOC-L-alanine and 20 mg of dimethylaminopyridine were added, and the solution was cooled to -10 °C. While stirring the solution, 177 mg of 1-ethyl-3-(3′-dimethylaminopropyl)-carbodiimide hydrochloride was added, and this was followed by 1 hour of agitation. The solution was further agitated at room temperature for 30 minutes, after which it was extracted with 50 ml of methylene chloride and 30 ml of 10% citric acid solution. The organic layer was washed with aqueous sodium bicarbonate and aqueous sodium chloride, and dried with anhydrous Glauber's salt. The solvent was concentrated under reduced pressure, the residue was subjected to silica gel column chromatography (benzeneethyl acetate =5:1), and the desired fraction was concentrated and solidified from isopropyl ether to thereby give 319 mg of the compound of Example 3 (5).

The physical data of the compound obtained here were the same as those of the compound of Example 3 (5). (2) 290 mg of the compound obtained in (1) was reacted in the same manner as in Example 3 (6) to thereby give 213 mg of pivaloyloxymethyl 7[D-O-(L-alanyl)mandelamido]-3-[(1,2,4-thiadiazol-5-yl)-thiomethyl]-3-cephem-4-carboxylate hydrochloride.

The physical data of the compound obtained here were the same as those of the compound of Example 3 (6).

Example 6

(5-Methyl-1,3-dioxol-2-on-4-yl)methyl 7-[D-O-(L-alanyl)mandelamido]-3-[(1,2,4-thiadiazol-5-yl)thiomethyl]-3-cephem-4-carboxylate hydrochloride

(1) To 65 ml of dimethylformamide, 10 g of the compound of Example 3 (1) was added. To this mixture, 5 ml of solution of 4-bromomethyl-5-methyl-1,3-dioxol-2-on in dimethylformamide was added at 20 °C while stirring the mixture, and this was followed by 3 hours of agitaion. The mixture was then extracted with 100 ml of 2% aqueous sodium chloride and 100 ml of ethyl acetate, and the organic layer was washed with sequential additions of water, aqueous sodium bicarbonate and aqueous sodium chloride. To the organic layer, 13.3 ml of methanol and 5.8 ml of concentrated hydrochloric acid were added, and this was followed by 1.5 hours of agitation at 22 °C. The separating crystal was collected by filtration and dried to thereby give 6.2 g of (5-methyl-1,3-dioxol-2-on-4-yl)methyl 7-amino-3-[(1,2,4-thiadiazol-5-yl)thiomethyl]-3-cephem-4-carboxylate hydrochloride.

I R (Nujol, $cm^{-1}$): 1825, 1795, 1740

N M R ($DMSO_{d\text{-}6}$, $\delta$ ppm): 2.20 (s, 3H, $-CH_3$), 3.82 (brs, 2H, 2-position $-H_2$), 4.30, 4.72 (ABq, J=13Hz, 2H, 3-position $-CH_2-$), 4.90~5.60 (m, 2H, 6-position -H, 7-position -H), 5.20 (s, 2H, $-OCH_2-$), 6.0~10.6 (br, 3H, $-NH_3^+$), 8.66 (s, 1H, thiadiazole 3-position -H).

2) To 4.5 g of the compound obtained in (1),15 ml of methylene chloride was added. This mixture was washed with aqueous sodium. bicarbonate and aqueous sodium chloride, and dried with anhydrous Glauber's salt. This solution was reacted in the same manner as in Example 4 to thereby give 5.4 g of the subject compound.

I R (Nujol, $cm^{-1}$): 1820, 1780, 1690, 1620

N M R ($DMSO_{d\text{-}6}$, $\delta$ ppm): 1.50 (d, J=7Hz, 3H, $\underline{CH_3}$ CHCO-), 2.19 (s, 3H, dioxole $-CH_3$), 3.64 (brs, 2H, 2-position $-H_2$), 3.85~4.85 (m, 3H, 3-position $-CH_2-$,

$CH_3$ C$\underline{H}$CO-) 5.5 (d, J=5Hz, 1H, 6-position -H), 5.09, 5.37 (d×d, J=12Hz, 13Hz, 2H, dioxole $-CH_2-$), 5.65, 5.78 (d×d, J=5Hz, 8Hz, 1H, 7-position -H), 6.11 (s, 1H, > C$\underline{H}$CONH-) 7.20~7.75 (m,5H, phenyl), 8.66 (s, 1H, thiadiazole 3-position -H), 8.4~9.1 (br, 3H, $-NH_3^+$), 9.42 (d, J=8Hz, 1H, -CONH-)

Example 7

1-Ethoxycarbonyloxyethyl 7-[D-O-(L-alanyl)mandelamido]-3-[(1,2,4-thiadiazol-5-yl)thiomethyl]-3-cephem-4-carboxylate hydrochloride

(1) To 4.0 g of sodium 7-D-mandelamido-3-(1,2,4-thiadiazol-5-yl)thiomethyl-3-cephem-4-carboxylate, 56 ml of a dimethylformamide-dimethylacetamide (1:1) mixture was added, and the resulting mixture was cooled to -30° C. While stirring the mixture, 2.78 g of α-iododiethyl carbonate in 8 ml of dimethylformamide-dimethylacetamide (1:1) mixture was added, and this was followed by reaction at -20° C for 1 hour and 40 minutes. The reaction liquid was extracted with 200 ml of cold 2% aqueous sodium chloride and 300 ml of cold ethyl acetate. The organic layer was washed with sequential additions of 2% aqueous sodium chloride and saturated aqueous sodium chloride, and dried with anhydrous Glauber's salt, after which it was concentrated under reduced pressure and solidified with isopropyl ether. The solid was subjected to silica gel column chromatography (benzen-ethyl acetate = 2:1), and the desired fraction was concentrated and crystallized from ethyl acetate-petroleum ether (1:1) mixture to thereby give 3.17 g of l-ethoxycarbonyloxyethyl 7-D-mandelamido-3-[(1,2,4-thiadiazol-5-yl)thiomethyl]-3-cephem-4-carboxylate.
I R (Nujol, $cm^{-1}$): 1785, 1760, 1735, 1690
N M R ($DMSO_{d\text{-}6}$, δ ppm): 1.18, 1.21 (t, t, J=7Hz, 3H, $-CH_2C\underline{H_3}$), 1.55 (d, J=6Hz, 3H, $>CHC\underline{H_3}$), 3.68 (s, 2H, 2-position $-H_2$), 3.85~4.85 (m, 4H, 3-position $-CH_2-$, $-C\underline{H_2}CH_3$), 5.9~5.3 (m, 2H, 6-position $-\underline{H}$, $>C\underline{H}OH$), 5.73 (d×d, J=5Hz, 8Hz, 1H, 7-position -H), 6.07, 6.09 (d, d, J=6Hz, 1H, -OH), 6.82, 6.88 (q, q, J=6Hz, 1H, $>C\underline{H}CH_3$), 7.1~7.6 (m, 5H, phenyl), 8.65 (s, 1H, thiadiazole 3-position -H), 8.69 (d, J=8Hz, 1H, -CONH-)

(2) 3.1 g of the compound obtained in (1) was reacted in the same manner as in Example 5 (1) to thereby give 3.70 g of 1-ethoxycarbonyloxyethyl 7-[D-O-(BOC-L-alanyl)mandelamido]-3-[(1,2,4-thiadiazol-5-yl)thiomethyl]-3-cephem-4-carboxylate.
I R (Nujol, $cm^{-1}$): 1790, 1760, 1710, 1685
N M R ($DMSO_{d\text{-}6}$, δ ppm): 1.0~1.5 (m, 6H, $-CH_2C\underline{H_3}$,

$C\underline{H_3}$ CHNH-), 1.35 (s, 9H, -BOC, 1.53 (d, J=6Hz, 3H, $>CHC\underline{H_3}$), 3.65 (brs, 2H, 2-position $-H_2$), 3.9~4.8 (m, 5H, 3-position $-CH_2-$, $-C\underline{H_2}CH_3$,

$CH_3$ $C\underline{H}NH-$), 5.05 (d, 5Hz, 1H, 6-position -H), 5.78 (d×d, J=5Hz, 8Hz, 1H, 7-position -H), 5.98 (s, 1H, $>C\underline{H}OH$), 6.83, 6.90 (q, q, J=6Hz, 1H, $>C\underline{H}CH_3$, 7.1~7.6 (m, 6H, phenyl,

$CH_3$ $CHN\underline{H}-$), 8.63 (s, 1H, thiadiazole 3-position -H), 9.26 (d, J=8Hz, 1H, -CONH-)

(3) 3.6 g of the compound obtained in (2) was reacted in the same manner as in Example 3 (6) to thereby give 2.79 g of the subject compound.
I R (Nujol, $cm^{-1}$): 1780, 1760, 1690
N M R ($DMSO_{d\text{-}6}$, δ ppm): 1.19, 1.22 (t, t, J=6Hz, 3H, $-CH_2C\underline{H_3}$), 1.48 (d, J=7Hz, 3H,

$C\underline{H_3}$ CHCO-), 1.54 (d, J=5Hz, 3H,

$C\underline{H_3}$ CHO- ), 3.65 (brs, 2H, 2-position $-H_2$), 4.08, 4.15 (q, q, J=6Hz, 2H, $-C\underline{H_2}CH_3$), 4.0~4.8 (m, 3H, 3-position $-CH_2-$,

$CH_3$ $C\underline{H}CO-$), 5.06 (d, J=5Hz, 1H, 6-position -H), 5.5~5.9 (m, 1H, 7-position -H), 6.10 (s, 1H, $>C\underline{H}CONH-$), 6.83, 6.89 (q, q, J=5Hz, 1H,

$CH_3$. $C\underline{H}O-$),7.1~7.7 (m, 5H, phenyl), 8.65, 8.67 (s, s, 1H, thiadiazole 3-position -H), 8.0~9.1 (br, 3H, $-NH_3^+$), 9.42 (d, J=8Hz, 1H, -CONH-)

The following compounds can be produced in accordance with any one of the methods of Example 3 through 7.

* Pivaloyloxymethyl 7-[D-O-(L-alanyl)mandelamido]-3-[(1,2,4-thiadiazol-5-yl)thiomethyl]-3-cephem-4-carboxylate
* Pivaloyloxymethyl 7-[D-O-(L-prolyl)mandelamido]-3-[(1,2,4-thiadiazol-5-yl)thiomethyl]-3-cephem-4-carboxylate hydrochloride
* Pivaloyloxymethyl 7-[D-O-(L-leucyl)mandelamido)]1-3-[(1,2,4-thiadiazol-5-yl)thiomethyl]-3-cephem-4-carboxylate hydrochloride

* Pivaloyloxymethyl 7-[D-O-(L-lysyl)mandelamido]-3-[(1,2,4-thiadiazol-5-yl)thiomethyl]-3-cephem-4-carboxylate dihydrochloride
* Pivaloyloxymethyl 7-[D-O-(glycyl)mandelamido]-3-[(1,2,4-thiadiazol-5-yl)thiomethyl]-3-cephem-4-carboxylate hydrochloride
* 1-Acetoxyethyl 7-[D-O-(L-alanyl)mandelamido]-3-[(1,2,4-thiadiazol-5-yl)thiomethyl]-3-cephem-4-carboxylate hydrochloride
* 1-Acetoxyethyl 7-[D-O-(L-prolyl)mandelamido]-3-[(1,2,4-thiadiazol-5-yl)thiomethyl]-3-cephem-4-carboxylate hydrochloride
* 1-Acetoxyethyl 7-[D-O-(L-leucyl)mandelamido]-3-[(1,2,4-thiadiazol-5-yl)thiomethyl]-3-cephem-4-carboxylate hydrochloride
* 1-Acetoxyethyl 7-[D-O-(L-lysyl)mandelamido]-3-[(1,2,4-thiadiazol-5-yl)thiomethyl]-3-cephem-4-carboxylate dihydrochloride
* 1-Acetoxyethyl 7-[D-O-(glycyl)mandelamido]-3-[(1,2,4-thiadiazol-5-yl)thiomethyl]-3-cephem-4-carboxylate hydrochloride
* 1-Ethoxycarbonyloxyethyl 7-[D-O-(L-prolyl)mandelamido]-3-[(1,2,4-thiadiazol-5-yl)thiomethyl]-3-cephem-4-carboxylate hydrochloride
* 1-Ethoxycarbonyloxyethyl 7-[D-O-(L-leucyl)mandelamido]-3-[(1,2,4-thiadiazole-5-yl)thiomethyl]-3-cephem-4-carboxylate hydrochloride
* 1-Ethoxycarbonyloxyethyl 7-[D-O-(L-lysyl)mandelamido]-3-[(1,2,4-thiadiazol-5-yl)thiomethyl]-3-cephem-4-carboxylate dihydrochloride
* 1-Ethoxycarbonyloxyethyl 7-[D-O-(glycyl)mandelamido]-3-[(1,2,4-thiadiazol-5-yl)thiomethyl]-3-cephem-4-carboxylate hydrochloride

Formulation Example 1

Tablets composed of the following ingredients are prepared by an ordinary method.

Compound of Example 3    125 mg potency
Polyvinylpyrrolidone    20 mg
Starch    20 mg
Magnesium stearate    2.0 mg

Formulation Example 2

Tablets composed of the following ingredients are prepared by an ordinary method.

Compound of Example 3    250 mg potency
Citric acid    50 mg
Starch    20 mg
Magnesium stearate    3.0 mg

Formulation Example 3

Tablets composed of the following ingredients are prepared by an ordinary method.

Compound of Example 3    500 mg potency
Starch    20 mg
Hydroxypropylcellulose    3mg
Magnesium stearate    5 mg

Formulation Example 4

The compound of Example 3 and tartaric acid are mixed together and an ordinary method of capsule packing is employed to prepare capsules.

Compound of Example 3  125 mg potency
Tartaric acid  25 mg
Magnesium stearate  1 mg
Add starch to an entire amount of  300 mg

Formulation Example 5

Capsules composed of the following ingredients are prepared in accordance with the method of Formulation Example 4.

Compound of Example 3  125 mg potency
Magnesium stearate  2 mg
Add lactose to an entire amount of  200 mg

Formulation Example 6

Dry syrup composed of the following ingredients is prepared.

Compound of Example 3  62.5 mg potency
Citric acid  25 mg
Sugar  70 mg
CMC-Na  20 mg

**Claims**

(1) A cephalosporin compound represented by the formula (I):

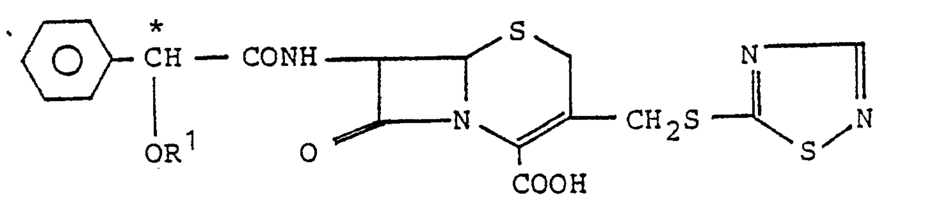

(I)

wherein $R^1$ represents a hydrogen atom or α-amino acid residue (ester bonding), its ester, or its pharmacologically acceptable salt.

(2) The compound as claimed in Claim (1) wherein $R^1$ is a hydrogen atom.

(3) The compound as claimed in Claim (1) wherein $R^1$ is an α-amino acid residue (ester bonding).

(4) The compound as claimed in Claim (3) wherein an α-amino acid residue is a neutral amino acid residue.

(5) The compound as claimed in Claim (1) wherein a carboxy group in the formula (I) forms as ester.

(6) The compound as claimed in Claim (5) wherein the hydrogen atom of the carboxy group in the formula (I) is substituted by an alkyl group, phthalidyl, 5-methyl-1,3-dioxol-2-on-4-ylmethyl or a group represented by the formula:

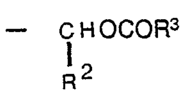

wherein R2 represents an alkyl group and R3 represents an alkyl group or alkoxy group.

(7) The compound as claimed in Claim (1) which is chosen from the compounds listed below.

* 7-D-(-)Mandelamido-3-(1,2,4-thiadiazol-5-yl)thiomethyl-3-cephem-4-carboxylic acid
* Pivaloyloxymethyl 7-D-(-)mandelamido-3-(1,2,4-thiadiazol-5-yl)thiomethyl-3-cephem-4-carboxylate

* Pivaloyloxymethyl 7-[D-O-(L-alanyl)mandelamido]-3-[(1,2,4-thiadiazole-5-yl)thiomethyl]-3-cephem-4-carboxylate hydrochloride
* (5-Methyl-1,3-dioxol-2-on-4-yl)methyl 7-[D-O-(L-alanyl)mandelamido]-3-[(1,2,4-thiadiazol-5-yl)thiomethyl]-3-cephem-4-carboxylate hydrochloride
* 1-Ethoxycarbonyloxyethyl 7-[D-O-(L-alanyl)mandelamido]-3-[(1,2,4-thiadiazol-5-yl)thiomethyl]-3-cephem-4-carboxylate hydrochloride

(8) The production method for a cephalosporin compound represented by the formula (I):

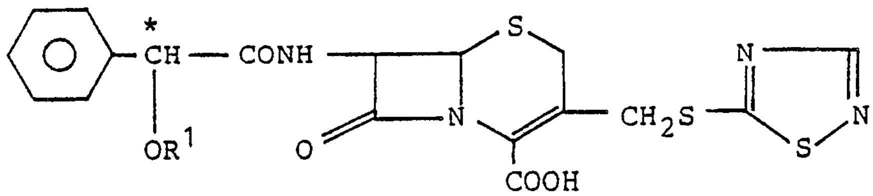

(I)

, its ester, or its pharmacologically acceptable salt, characterized by:

(a) reaction between a compound represented by the formula (II):

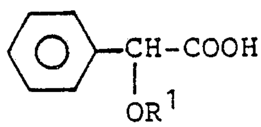

(II)

or the formula (II-1):

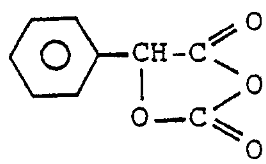

(II-1)

and a compound represented by the formula (III):

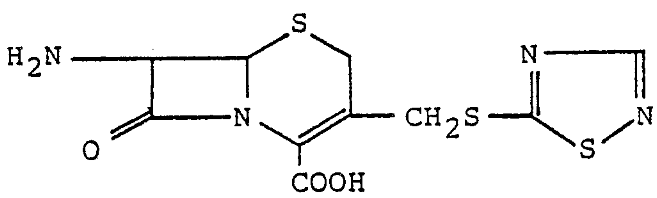

(III)

, its ester or its salt;

(b) nucleophilic substitution reaction of 3-position Y of a compound represented by the formula (VI):

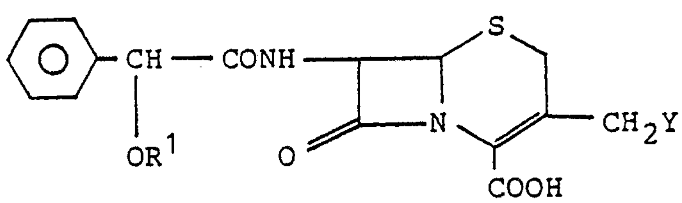

(VI)

, its ester or its salt, with 1,2,4-thiadiazol-5-thiol;

(c) reaction between a compound represented by the formula (I-1):

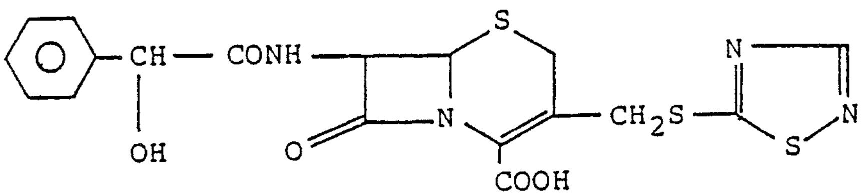

(I-1)

. its ester or its salt and a compound represented by the formula (VIII):

$R^5$—OH

; or (d) reaction between a compound represented by the formula (I-2):

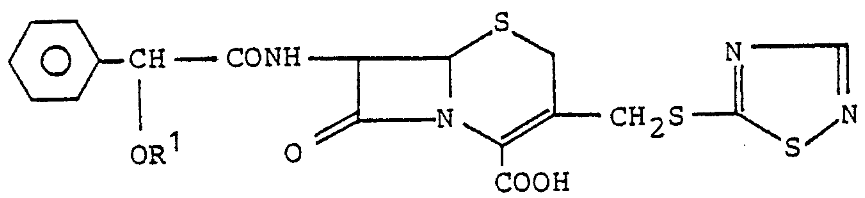

(I-2)

and a compound represented by the formula (V):

X—$R^4$

In the above formulae, $R^1$ represents a hydrogen atom or $\alpha$-amino acid residue (ester bonding); $R^4$ represents a group introduced by the esterification; $R^5$ represents an $\alpha$-amino acid residue; X represents a carboxy-reactive group such as halogen, alkylsulfonate or arylsulfonate; and Y represents a nucleophilic residue.

(9) A composition for prevention and treatment of bacterial infections, comprising an effective amount of at least one compound selected from the cephalosporin compound as claimed in Claim (1), its ester or its pharmacologically acceptable salt, and one or more pharmaceutically acceptable additives.

(10) A method for treatment of bacterial infectious diseases caused by Gram-positive bacteria or Gram-negative bacteria, which comprises administering of an effective amount of a cephalosporin compound as claimed in Claim 1.